# EUROPÄISCHE PATENTSCHRIFT

(11) **EP 1 022 267 B1**
(45) Veröffentlichungstag und Bekanntmachung des Hinweises auf die Patenterteilung: **06.04.2005**
(21) Anmeldenummer: 00100128.8
(22) Anmeldetag: 07.01.2000
(51) Int. Cl.: C07C 245/20, C07B 63/00, C09B 67/54, C09B 41/00

(54) **Verfahren zur Herstellung von natriumionenarmen Diazoniumverbindungen**
Process for the manufacture of diazonium compounds with a low sodium ions content
Procédé de préparation de composés de diazonium ayant une faible teneur en ions sodium

(30) Priorität: 20.01.1999 US 234449
(43) Veröffentlichungstag der Anmeldung: 26.07.2000
(73) Patentinhaber: Bayer Chemicals AG, 51368 Leverkusen (DE); Bayer Corporation, Pittsburgh, PA 15205-9741 (US)
(72) Erfinder: Dubose, John C., Summerville, SC 29485 (US); Johnson, Andrew D., Summerville, SC 29485 (US); Murphree, Samuel Shaun, Meadville, PA 16335 (US); Tholema, Edzard, Dr., 51519 Odenthal (DE); Hoffmann, Helmut, 51377 Leverkusen (DE); Zarges, Wolfgang, Dr., 51069 Köln (DE); Frank, Wolfgang, Dr., 50939 Köln (DE); Burkhardt, Bettina, Dr., 47799 Krefeld (DE)
(74) Vertreter: Herbold, Matthias

(56) Entgegenhaltungen:
- EP-A- 0 652 044
- FR-A- 2 289 579
- GB-A- 2 015 018
- US-A- 4 877 412
- US-A- 5 173 086
- US-A- 5 282 939

## Beschreibung

Die Erfindung betrifft ein Verfahren zur Herstellung von natriumionenarmen Diazoniumverbindungen sowie die Verwendung so hergestellter Verbindungen zur Herstellung von Azoverbindungen, insbesondere Azofarbstoffen.

Für die meisten Farbstoffeinsätze werden Farbstoffe mit hoher Wasserlöslichkeit gefordert. Bestimmte Salzformen von sulfo- oder carboxygruppenhaltigen Farbstoffen sind beispielsweise schwerer wasserlöslich als andere. Besonders schwerlöslich sind häufig die entsprechenden Na-Salze oder Natrium-Mischsalze, wie die Na/K-Salze. Erstrebenswert ist es daher, möglichst die Farbstoffe anderer Salzformen, wie beispielsweise die Lithium-, Kalium- oder Ammoniumsalze bereitzustellen. Insbesondere bei relativ schlecht löslichen Farbstoffsalzen ist eine Umsalzung in die entsprechenden besser wasserlöslichen Salzformen sehr umständlich, da mit hoher Verdünnung gearbeitet werden muß und ein erheblicher Abwasseranfall verursacht wird.

Daher versucht man bereits für die Kupplungsreaktion zur Herstellung entsprechender Azoverbindungen, insbesondere Azofarbstoffen, natriumionenarme bzw. -freie Ausgangsstoffe einzusetzen.

Die Diazoniumverbindungen, die im allgemeinen durch Diazotierung aus den entsprechenden Aminen mit Natriumnitrit in Gegenwart von anorganischen Säuren hergestellt werden, fallen oft als wasserunlösliche Verbindungen an, die abfiltriert und dann mit Wasser frei von Natriumionen gewaschen werden können. Dieses Verfahren ist sicherheitstechnisch bedenklich, da der Filterkuchen antrocknen kann und bei Schlag- oder Wärmeeinwirkung dann eine explosionsartige Zersetzung der bekanntermaßen im trochenen Zustand instabilen Diazonium-Verbindungen zu erwarten ist.

Es wurde nun ein Verfahren gefunden, bei dem die oben beschriebenen Nachteile vermieden werden.

Das erfindungsgemäße Verfahren betrifft die Entsalzung wäßriger Suspensionen, enthaltend wenigstens eine Diazoniumverbindung und natriumionenhaltige Elektrolyte, dadurch gekennzeichnet, daß die wäßrige Suspension über eine semipermeable Membran permeiert wird. Die Verbindungen können auch als Betain vorliegen.

Als bevorzugte Diazoniumverbindungen werden aromatische Diazoniumverbindungen eingesetzt, die besonders bevorzugt eine oder mehrere Sulfo- und/oder Carboxygruppen tragen. Derartige Diazoniumverbindungen besitzen vorzugsweise eine Wasserlöslichkeit von <5, insbesondere <1 g/l bei 20°C. Als Gegenanionen sind Halogenide, insbesondere Chlorid sowie Sulfat bevorzugt. Die Verbindungen können aber auch als Betain vorliegen.

Besonders bevorzugte Diazoniumverbindungen sind solche, ausgewählt aus der Gruppe der Formeln (I) und (II) 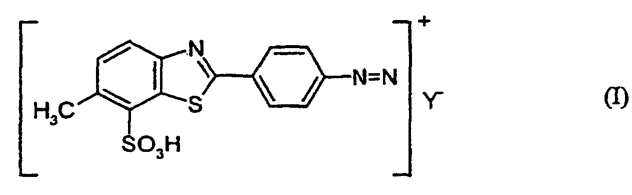 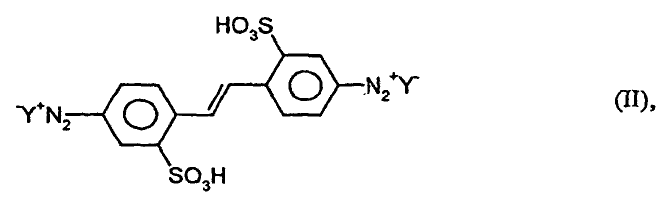 worin
- Y⁻: für ein Anion, vorzugsweise Halogenid und Sulfat, insbesondere für Cl⁻ und (SO²⁻₄)_{1/2} steht,
oder deren Betaine.

Die für das erfindungsgemäße Verfahren eingesetzten Suspensionen besitzen vorzugsweise einen pH-Wert von 0,5 bis 5, insbesondere 1 bis 3. Die membrantechnische Entsalzung wird vorzugsweise bei einer Temperatur von 5 bis 70°C, insbesondere bei 20 bis 50°C durchgeführt. Bei dem erfindungsgemäßen Verfahren werden insbesondere natriumionenhaltige Elektrolyte wie NaCl, Na₂SO₄, NaHSO₄ sowie NaOAc abgetrennt. Bevorzugt werden auch weitere anorganische Elektrolyte abgetrennt, soweit vorhanden. Bevorzugt haben die eingesetzten wäßrigen Suspensionen Natriumionengehalte von 0,2 bis 5,0 Gew.-%. Im Anschluß an die membrantechnische Entsalzung nach dem erfindungsgemäßen Verfahren besitzen die wäßrigen Suspensionen einen Natriumionengehalt von vorzugsweise weniger als 0,1 %, vorzugsweise weniger als 0,05 %. Bevorzugt gelten diese Werte auch für den Gesamtgehalt an anorganischen Elektrolyten.

Im übrigen wird unter natriumionenarm bzw. elektrolytarm im Rahmen dieser Anmeldung ein Na⁺- bzw. Elektrolytgehalt von weniger als 0,1 Gew.-%, bezogen auf die Suspension verstanden.

Die in dem erfindungsgemäßen Verfahren eingesetzten wäßrigen Suspensionen entsprechen bevorzugt den Reaktionssuspensionen, wie sie durch Umsetzung der entsprechenden Amine mit Alkalinitrit in anorganischen Säuren, vorzugsweise Salzsäure und/oder Schwefelsäure erhalten werden.

Die im erfindungsgemäßen Verfahren eingesetzten semipermeablen Membranen sind vorzugsweise aus anorganischen oder organischen Materialien hergestellt. Als solche kommen beispielsweise Keramik oder säureresistente organische Polymerwerkstoffe in Frage. Die membrantechnische Entsalzung des erfindungsgemäßen Verfahrens erfolgt im allgemeinen nach der sogenannten cross-flow-Filtrationsmethode.

Unter cross-flow-Filtration wird verstanden, daß der Produktstrom tagential über die Membranoberfläche strömt, wobei der Aufbau von Deckschichten oder Filterkuchen aufgrund der erzeugten Scherkräfte vermieden wird.

Besonders bevorzugt kommen bei dem erfindungsgemäßen Verfahren Mikro- sowie Ultrafiltrationsmembranen zur Anwendung, vorzugsweise in Tubular- oder Kapillarmodulbauform. Als weitere mögliche Membranbauformen sind die Spiralwickel- und Kissenmodule zu nennen.

Die Membranen werden vorzugsweise aus anorganischen (z.B. TiO₂, ZrO₂, Al₂O₃) oder organischen Werkstoffen wie z.B. Polypropylen, teil- oder vollfluorierte Polymere, Polyhydantoin (siehe DE-A-43 38 196), Polysulfon sowie Polyamid hergestellt. Bevorzugt werden Membranen mit einer Trenngrenze von 3.000 bis 200.000 Dalton, vorzugsweise 50.000 bis 200.000 Dalton eingesetzt. Als solche kommen insbesondere Ultrafiltrationsmembranen zum Einsatz. Ebenfalls bevorzugte Membranen besitzen Porenweiten von 0,01 bis 10 µm, vorzugsweise 0,02 bis 0,2 µm. Als solche Membranen werden bevorzugt Mikrofiltrationsmembranen eingesetzt.

Das erfindungsgemäße Entsalzungsverfahren kann dabei entweder durch Aufkonzentration betrieben werden, wobei der Suspension Wasser entzogen wird oder durch Diafiltration betrieben werden, wobei die membrantechnisch entnommene Menge an Permeat durch entsalztes, insbesondere natriumarmes bzw. -freies entionisiertes Wasser wieder zugeführt wird.

Bevorzugt werden die nach dem erfindungsgemäßen Verfahren erhaltenen wäßrigen Suspensionen von Diazoniumverbindungen in Gegenwart einer insbesondere natriumfreien Base zur Herstellung einer Azoverbindung, insbesondere eines Azofarbstoffes eingesetzt. Als besonders bevorzugte Kupplungskomponenten kommen dabei Barbitursäure bzw. deren Derivate, Acetessigsäurederivate und Pyrazolone, insbesondere für die Umsetzung der Diazoniumverbindung der Formel (I) zur Herstellung von Gelbfarbstoffen in Frage sowie H-Säure und Salicylsäurederivate, wie z.B. 3-Methyl-2-hydroxybenzoesäure, insbesondere für die Umsetzung mit der Diazoniumverbindung der Formel (II) zur Herstellung von Blau + Gelbfarbstoffen in Frage. Als bevorzugte natriumionenfreie Basen zur Herstellung von Farbstoffen unter Verwendung der nach dem erfindungsgemäßen Verfahren entsalzten wäßrigen Suspensionen sind beispielsweise LiOH, KOH, (CH₃)₄NOH sowie Amine, insbesondere Alkanolamine wie beispielsweise CH₃-NH-CH₂CH₂OH, CH₃-N(CH₂CH₂OH)₂, H₂N-CH₂CH₂OH, HN(CH₂CH₂OH)₂, N(CH₂CH₂OH)₃, (CH₃CH₂)₂NCH₂CH₂OH, N-(-CH₂-CH₂-O-CH₂-CH₂-OH)₃, 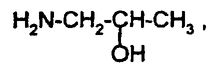 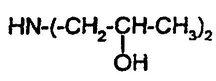 oder 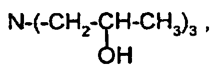 und Mischungen derselben. Besonders bevorzugt sind tertiäre Amine. Die entsprechenden Farbstoffe eignen sich insbesondere zum Färben von cellulosehaltigen Materialien, vorzugsweise Papier, Baumwolle und Viskose sowie zum Inkjet-Druck.

### Beispiele

### Beispiel 1

1,00 Moläquivalent Dehydrothiotoluidinsäure wird mit 0,99 Moläquivalent Natriumnitrit und 1,65 Moläquivalent Salzsäure zum Diazoniumion (I) umgesetzt (Y = Cl⁻).

72 kg dieser Synthesesuspension (pH 1,2; Natriumgehalt 2800 ppm; Gehalt Diazoniumion 0,13 mol/kg) werden bei 18°C einer Querstrom-Ultrafiltration unterzogen. Zur Anwendung kommt dabei eine Polymermembran (Polyhydantoin auf Polyphenylensulfidträger) als ½"-Tubularmembran, wie sie z.B. in EP-A 652 044 offenbart wurde. Das verwendete Modul hat bei einer Länge von 1,2 m eine Membranfläche von insgesamt 0,9 m².

### Entsalzung/Aufkonzentrierung

Beginnend bei einem Moduleingangsdruck von 10 bar werden zunächst 42 kg Permeat abgezogen, bevor durch Abzug von 180 kg Permeat und kontinuierliche Zugabe von 180 kg vollentsalztem Wasser diafiltriert wird. Dabei hebt man den Moduleingangsdruck auf 20 bar an.

Durch Entfernung von 16 kg Permeat wird endaufkonzentriert. Die Permeatstromdichte beträgt anfangs ca. 230 kg/(m²h) und bei der abschließenden Autkonzentrierung noch etwa 110 kg/(m²h).

Das Retentat hat einen Natriumgehalt von 90 ppm bei einem Gehalt des Diazoniumions von 0,66 mol/kg.

### Kupplungsreaktion

Die so erhaltene natriumarme Diazoniumsuspension wird mit 0,96 Moläquivalenten Barbitursäure, 0,17 Moläquivalenten LiOH und 0,79 Moläquivalenten Triethanolamin zum Zielfarbstoff der Formel (III) 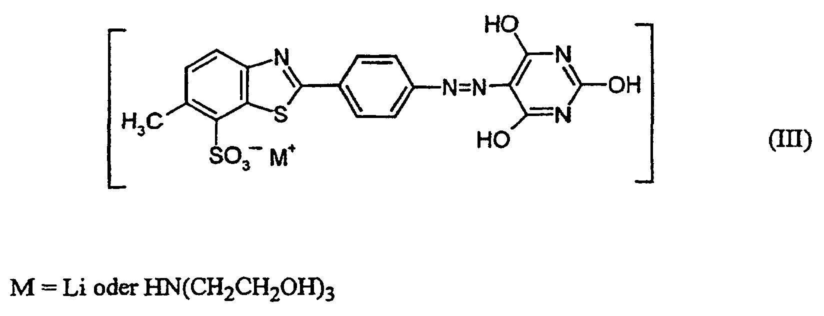 umgesetzt. Nach Standardisierung (Verdünnung mit vollentsalztem Wasser auf spezifizierten Farbstoffgehalt und Konservierung) erhält man eine lagerstabile, hochkonzentrierte Flüssigformulierung.

### Beispiel 2

Bei der Bearbeitung der Diazoniumionsuspension (I) kann alternativ auch eine keramische Mikrofiltrationsmembran eingesetzt werden: Eine Kapillarmembran der Fa. Membralox (ZrO₂ als aktive Trennschicht) mit einer Porenweite von 0,05 µm (Kanalhöhe 6 mm, Membranfläche 0,9 m²) wurde bei einem Moduleingangsdruck von 2 bar und einer Temperatur von 45°C betrieben.

Nach der anfänglichen Aufkonzentrierung auf 60 % des Startvolumens (ca. 950 l) wird das Gesamtvolumen 5,2 mal gegen vollentsalztes Wasser ausgetauscht, bis die Leitfähigkeit der Suspension einen Wert von 500 µS/cm erreicht. Der Natriumgehalt im Retentat beträgt 35 ppm. Die durchschnittliche Permeatflußdichte beträgt 185 l/(m²h).

Die so erhaltene natriumarme Diazoniumionsuspension wird mit Cyaniminobarbitursäure und Triethanolamin zum gelben Zielfarbstoff der Formel (IV) 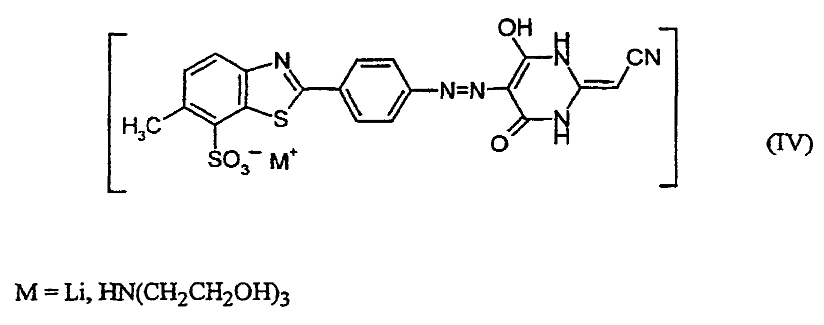 umgesetzt. Nach Standardisierung (Einstellung auf festgelegten Farbstoffgehalt, Konservierung) erhält man eine lagerstabile hochkonzentrierte Flüssigformulierung.

## Patentansprüche

1. Verfahren zur Entsalzung wässriger Suspensionen, enthaltend eine organische Diazoniumverbindung und Na-ionenhaltige Elektrolyte, **dadurch gekennzeichnet, dass** die wässrige Suspension über eine semipermeable Membran permeiert wird.

2. Verfahren gemäß Anspruch 1, **dadurch gekennzeichnet, dass** als semipermeable Membranen Mikro- oder Ultrafiltrationsmembranen eingesetzt werden.

3. Verfahren gemäß Anspruch 1, **dadurch gekennzeichnet, dass** das membrantechnische Verfahren nach der sogenannten cross-flow-Filtration betrieben wird.

4. Verfahren gemäß Anspruch 1, **dadurch gekennzeichnet, dass** als organische Diazoniumverbindungen solche der Formeln (I) und (II) 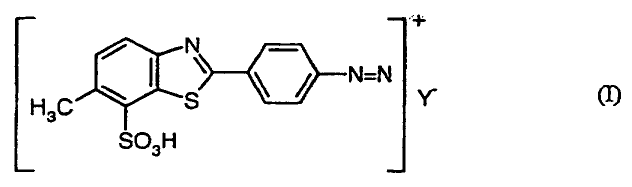 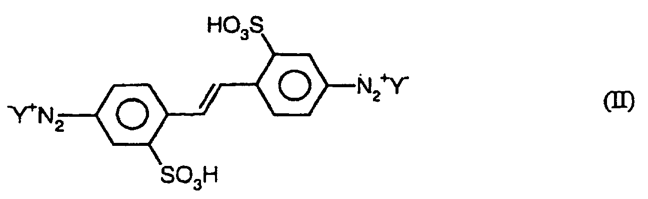 worin
Y- für ein Anion, vorzugsweise Halogenid und Sulfaten, insbesondere für Cl⁻ und (SO²⁻₄)_{1/2} steht,
oder deren Betaine eingesetzt werden.

5. Verfahren gemäß Anspruch 1, **dadurch gekennzeichnet, dass** die Entsalzung der Suspension bei pH-Werten von 0,5 bis 5, insbesondere von 1 bis 3 durchgeführt wird.

6. Verfahren gemäß Anspruch 1, **dadurch gekennzeichnet, dass** die Entsalzung der wässrigen Suspension bei einer Temperatur von 5 bis 70, vorzugsweise 20 bis 50°C durchgeführt wird.

7. Verfahren nach Anspruch 1, **dadurch gekennzeichnet, dass** die erhaltenen wässrigen Suspensionen organischer Diazoniumverbindungen zur Herstellung von Azoverbindungen, vorzugsweise Azofarbstoffen eingesetzt werden.

8. Verfahren nach Anspruch 7, **dadurch gekennzeichnet, dass** die Kupplungsreaktion in Gegenwart einer oder mehrerer organischen natriumionenfreien Base durchgeführt wird.

9. Verfahren nach Anspruch 7, **dadurch gekennzeichnet, dass** als Kupplungskomponenten Barbitursäure, Barbitursäure-Derivate, Acetessigsäure oder -amide, Salicylsäure, Salicylsäure-Derivate oder Naphthalinsulfonsäurederivate eingesetzt werden.

10. Verfahren gemäß Anspruch 7, **dadurch gekennzeichnet, dass** die erhaltenen Azoverbindungen zum Färben von cellulosehaltigen Materialien, insbesondere Papier, Baumwolle, Viskose sowie zum Inkjet-Druck eingesetzt werden.

## Claims

1. Process for desalting aqueous suspensions containing an organic diazonium compound and electrolytes containing sodium ions, **characterized in that** the aqueous suspension is permeated through a semipermeable membrane.

2. Process according to Claim 1, **characterized in that** the semipermeable membranes used are microfiltration or ultrafiltration membranes.

3. Process according to Claim 1, **characterized in that** the membrane process is carried out by means of crossflow filtration.

4. Process according to Claim 1, **characterized in that** the organic diazonium compounds used are those of the formulae (I) and (II) 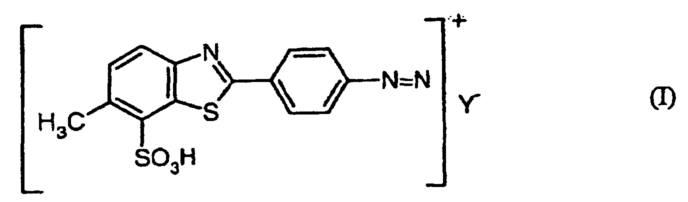 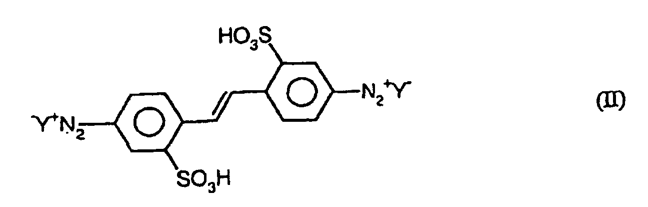 wherein Y⁻ represents an anion, preferably halide and sulphates, in particular Cl⁻ and (SO²⁻₄)_{1/2}, or betaines thereof.

5. Process according to Claim 1, **characterized in that** the desalination of the suspension is carried out at pH values of 0.5 to 5, in particular of 1 to 3.

6. Process according to Claim 1, **characterized in that** the desalination of the aqueous suspension is carried out at a temperature of 5 to 70°C, preferably 20 to 50°C.

7. Process according to Claim 1, **characterized in that** the aqueous suspensions of organic diazonium compounds obtained are used for the preparation of azo compounds, preferably azo dyes.

8. Process according to Claim 7, **characterized in that** the coupling reaction is carried out in the presence of one or more organic bases free of sodium ions.

9. Process according to Claim 7, **characterized in that** the coupling components used are barbituric acid or derivatives thereof, acetoacetic acid or amides thereof, salicylic acid or derivatives thereof, or naphthalene-sulphonic acid derivatives.

10. Process according to Claim 7, **characterized in that** the azo compounds obtained are used for dyeing cellulosic materials, in particular paper, cotton, viscose and for ink jet printing.

## Revendications

1. Procédé pour dessaler des suspensions aqueuses contenant un dérivé organique de diazonium et des électrolytes contenant eux-mêmes des ions Na, **caractérisé en ce que** l'on soumet la suspension aqueuse à perméation sur une membrane semiperméable.

2. Procédé selon la revendication 1, **caractérisé en ce que** les membranes semiperméables utilisées sont des membranes de microfiltration ou d'ultrafiltration.

3. Procédé selon la revendication 1, **caractérisé en ce que** l'opération technique sur membrane est réalisée sous la forme d'une filtration en "cross-flow".

4. Procédé selon la revendication 1, **caractérisé en ce que** les dérivés organiques de diazonium en question sont des composés de formules (I) et (II) 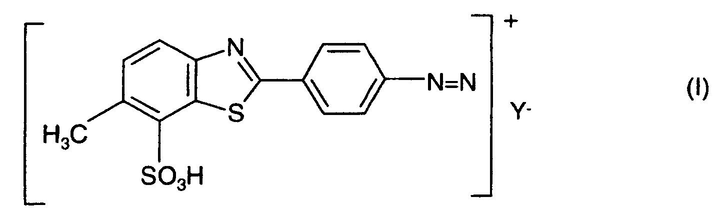 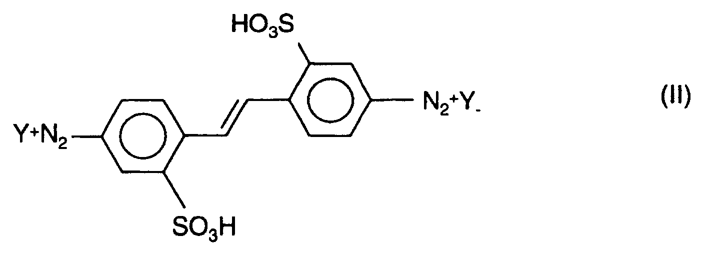 dans lesquelles
Y- représente un anion, de préférence un anion halogénure ou sulfate, plus spécialement un anion Cl⁻ ou (SO₄⁻²)_{1/2},
ou leurs bétaïnes.

5. Procédé selon la revendication 1, **caractérisé en ce que** le dessalage de la suspension est réalisé à des pH de 0,5 à 5, et plus spécialement de 1 à 3.

6. Procédé selon la revendication 1, **caractérisé en ce que** le dessalage de la suspension aqueuse est réalisé à une température de 5 à 70, de préférence de 20 à 50°C.

7. Procédé selon la revendication 1, **caractérisé en ce que** les suspensions aqueuses de dérivés organiques de diazonium obtenues sont elles-mêmes utilisées pour la préparation de dérivés azoïques, de préférence des colorants azoïques.

8. Procédé selon la revendication 7, **caractérisé en ce que** la réaction de copulation est réalisée en présence d'une ou plusieurs bases organiques exemptes d'ions sodium.

9. Procédé selon la revendication 7, **caractérisé en ce que** l'on utilise en tant que copulants l'acide barbiturique, des dérivés de l'acide barbiturique, l'acide acétylacétique ou des acétylacétamides, l'acide salicylique, des dérivés de l'acide salicylique ou des dérivés d'acides naphtalènesulfoniques.

10. Procédé selon la revendication 7, **caractérisé en ce que** les dérivés azoïques obtenus sont utilisés pour la teinture de matières contenant de la cellulose, en particulier le papier, le coton, la viscose, ou pour l'impression par jet d'encre.
